# EUROPÄISCHE PATENTSCHRIFT

(11) **EP 1 208 081 B1**
(45) Veröffentlichungstag und Bekanntmachung des Hinweises auf die Patenterteilung: **14.04.2004**
(21) Anmeldenummer: 00964050.9
(22) Anmeldetag: 29.08.2000
(51) Int. Cl.: C07C 253/30, C07C 255/19

(54) **VERFAHREN ZUR HERSTELLUNG VON CYANESSIGSÄUREESTERN**
METHOD FOR PRODUCING CYANOACETIC ACID ESTERS
PROCEDE DE FABRICATION D'ESTERS D'ACIDE CYANACETIQUE

(30) Priorität: 30.08.1999 EP 99117033; 28.02.2000 US 185372 P
(43) Veröffentlichungstag der Anmeldung: 29.05.2002
(73) Patentinhaber: Lonza AG, 4052 Basel (CH)
(72) Erfinder: HANSELMANN, Paul, CH-3902 Brig-Glis (CH); HILDBRAND, Stefan, CH-4125 Riehen (CH)
(86) Internationale Anmeldenummer: PCT/EP2000/008397
(87) Internationale Veröffentlichungsnummer: WO 2001/016092

(56) Entgegenhaltungen:
- WO-A-92/12962
- US-A- 4 438 041
- WERMECKES, B. ET AL: "Anodic oxidation of.beta.-cyanoethyl ethers" ELECTROCHIM. ACTA, Bd. 30, Nr. 11, 1985, Seiten 1491-1500, XP000978657 in der Anmeldung erwähnt
- WERMECKES, BERND ET AL: "Anodic oxidation of 2-cyanoethanol to cyanoacetic acid" CHEM. BER. (1985), 118(9), 3771-80 , 1985, XP000979123

## Beschreibung

Die Erfindung betrifft ein Verfahren zur Herstellung von Cyanessigsäureestern der allgemeinen Formel 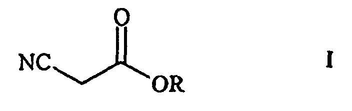 ausgehend von einem Alkoxypropionitril.
Der Rest R bedeutet hier und im folgenden eine lineare oder verzweigte C₁₋₈-Alkylgruppe. C₁₋₈-Alkylgruppen sind beispielsweise Methyl, Ethyl, Propyl, Isopropyl, Butyl, Isobutyl, *tert*-Butyl, Butyl, Pentyl, Hexyl, 2-Ethylbutyl, Heptyl, Octyl oder 2-Ethylhexyl.

Cyanessigsäureester der allgemeinen Formel I sind wichtige Ausgangsmaterialien für die Synthese von organischen Verbindungen, beispielsweise von pharmazeutischen Wirkstoffen.

Bekannt ist die elektrochemische Oxidation von Alkyl-β-cyanethylethern (Alkoxypropionitrilen) mit Platin oder Bleioxid als Anode in wässriger Schwefelsäure (B. Wermeckes, F. Beck, *Electrochim*. *Acta* **1985**, *30*, 1491). Dabei werden als Hauptprodukte nicht die hier gewünschten Ester, sondern Cyanessigsäure und die der Alkylgruppe entsprechende Carbonsäure wie z. B. Ameisensäure oder Essigsäure erhalten.

WO 92/12962 beschreibt die Oxidation von partiell oxidierten Propionitrilen (beispielsweise Cyanacetaldehyd oder dessen Acetale) mit Sauerstoff und anderen Oxidationsmitteln in Gegenwart von Katalysatoren wie Eisenchlorid oder Palladiumchlorid, wobei ebenfalls zunächst Cyanessigsäure gebildet wird, die dann durch säurekatalysierte Veresterung mit dem entsprechenden Alkohol in einen Cyanessigsäureester übergeführt werden kann.

US 4 438 041 offenbart die Herstellung von Cyanessigsäureestern durch Umsetzung von Cyanacetaldehydacetalen mit Hydroxylamin und einem Alkohol, gegebenenfalls in Gegenwart einer Säure.

Aufgabe der vorliegenden Erfindung war es, ein alternatives Verfahren zur Herstellung von Cyanessigsäureestem zur Verfügung zu stellen, bei dem die gewünschten Cyanessigsäureester direkt gebildet werden.

Diese Aufgabe wird mit dem Verfahren gemäss Patentanspruch 1 gelöst.

Überraschenderweise wurde gefunden, dass bei der Oxidation von Alkoxypropionitrilen der allgemeinen Formel 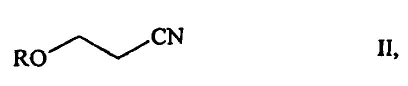 worin R die oben genannte Bedeutung hat, mit Sauerstoff oder einem Sauerstoff bildenden Reagens in Gegenwart einer Cobalt verbindung als Katalysator die Cyanessigsäureester der allgemeinen Formel I direkt gebildet werden.

Je nach der Bedeutung von R und dem herzustellenden Ester können als Edukte beispielsweise 3-Methoxypropionitril, 3-Ethoxypropionitril, 3-Propoxypropionitril, oder 3-Butoxypropionitril eingesetzt werden. Diese Edukte sind entweder käufliche Verbindungen, oder können nach bekannten Verfahren (s. z. B.: B. Wermeckes, F. Beck, *Electrochim*. *Acta* **1985**, *30*, 1491) hergestellt werden, beispielsweise durch Addition des entsprechenden Alkohols an Acrylnitril.

Als Sauerstoff bildendes Reagens kann beispielsweise Wasserstoffperoxid verwendet werden.

Als Katalysator für die Oxidation wird eine Cobaltverbindung wie beispielsweise Cobalt(II)acetat-Tetrahydrat oder Cobaltacetylacetonat eingesetzt.

Besonders bevorzugt ist Cobalt(II)acetat-Tetrahydrat.
Der Katalysator wird vorzugsweise in einer Menge von 0,01 bis 10 mol%, besonders bevorzugt in einer Menge von 0,01 bis 3 mol% eingesetzt.

Vorteilhaft wird die Oxidation bei einer Temperatur von 50 bis 250 °C, vorzugsweise bei einer Temperatur von 100 bis 200 °C durchgeführt.

Üblicherweise wird die Oxidation unter Druck durchgeführt, beispielsweise bei 5 bis 15 bar.

Die Oxidation wird entweder ohne Lösungsmittel oder in einem anorganischen oder organischen Lösungsmittel durchgeführt. Als organisches Lösungsmittel kann beispielsweise Acetonitril, Essigsäure, Toluol, Ethylacetat, Aceton, Tetrahydrofuran oder ein Alkohol wie Methanol, Ethanol, Propanol oder Butanol eingesetzt werden. Als anorganisches Lösungsmittel kann beispielsweise Wasser verwendet werden. Vorzugsweise wird ein organisches Lösungsmittel wie Acetonitril eingesetzt.

Vorteilhaft wird die Oxidation in Gegenwart eines Radikalbildners wie beispielsweise *N*-Hydroxyphthalimid, *N*-Hydroxysuccinimid oder *N*-Hydroxymaleinimid durchgeführt.

Die folgenden Beispiele verdeutlichen die Durchführung des erfindungsgemässen Verfahrens, ohne dass darin eine Einschränkung zu sehen ist.

### Beispiel 1

### Cyanessigsäuremethylester

**1.1** Auf eine Lösung von 3,0 g (0,035 mol) 3-Methoxypropionitril, 0,5 g (3,0 mmol) *N*-Hydroxyphthalimid (NHPI) und 57 mg (0,23 mmol) Co(CH₃COO)₂·4 H₂O in 50 ml Essigsäure wurden 9 bar O₂ in einem Autoklaven aufgepresst. Das Gemisch wurde anschliessend 5 h bei 150 °C gerührt. Der Autoklav wurde entspannt und das Reaktionsgemisch gaschromatographisch analysiert. Die Bestimmung der Edukt/Produkt-Verhältnisse erfolgte durch Vergleich der jeweiligen Peakflächen. Als Gerät diente ein HP 5890 Gaschromatograph mit WLD-Detektor und Permabond® Carbowax-Säule. Gefunden wurde ein Verhältnis 3-Methoxypropionitril/Cyanessigsäuremethylester von 11:1.
**1.2** Auf eine Lösung von 3,0 g (0,035 mol) 3-Methoxypropionitril, 1,0 g (6,13 mmol) NHPI und 112 mg (0,45 mmol) Co(CH₃COO)₂·4 H₂O in 30 g Acetonitril wurden 9 bar O₂ aufgepresst. Das Gemisch wurde 7 h bei 190°C gerührt. Der Autoklav wurde entspannt und das Reaktionsgemisch gaschromatographisch entsprechend Beispiel 1.1 analysiert. Gefunden wurde ein Verhältnis 3-Methoxypropionitril/Cyanessigsäuremethylester von 2:1. Die Reaktion wurde anschliessend aufgearbeitet: Acetonitril wurde am Rotationsverdampfer abdestilliert, der Rückstand in 50 ml Diethylether aufgenommen und filtriert. Das Filtrat wurde mit Wasser (50 ml) gewaschen, das Lösungsmittel am Rotationsverdampfer abgezogen und der Rückstand mittels Flash-Säulenchromatographie (Hexan/Essigsäureethylester 3:1) gereinigt. Die vollständige Abtrennung des Edukts gelang nicht.
   ¹H-NMR (CDCl₃, 300 MHz) δ: 3,81 (s, 3H); 3,5 (s, 2H).
**1.3** Auf eine Lösung von 1,0 g (6,13 mmol) NHPI und 100 mg (0,40 mmol) Co(CH₃COO)₂·4 H₂O in 20,0 g (0,23 mol) 3-Methoxypropionitril wurden 9 bar O₂ aufgepresst. Das Gemisch wurde 3 h bei 130 °C gerührt und anschliessend der Autoklav entspannt. Gemäss GC entsprechend zu Beispiel 1.1 wurde ein Verhältnis von 9:1 (Edukt/Cyanessigsäuremethylester) erhalten.
**1.4** Auf eine Lösung von 6,0 g (0,07 mol) 3-Methoxypropionitril, 2,5 g (0,015 mol) NHPI und 0,5 g (2 mmol) Co(CH₃COO)₂·4 H₂O in 30 g Acetonitril wurden 9 bar O₂ aufgepresst. Das Gemisch wurde 5,5 h bei 180 °C gerührt. Der Autoklav wurde entspannt und das Reaktionsgemisch gaschromatographisch entsprechend zu Beispiel 1.1 analysiert. Es wurde ein Verhältnis Edukt/Cyanessigsäuremethylester von 5:1 gefunden.

### Beispiel 2

### Cyanessigsäureethylester

Auf eine Lösung von 3,0 g (0,030 mol) 3-Ethoxypropionitril, 1,0 g (6,13 mmol) NHPI und 116 mg (0,47 mmol) Co(CH₃COO)₂·4 H₂O in 30 g Acetonitril wurden 9 bar O₂ aufgepresst. Das Gemisch wurde 7 h bei 190 °C gerührt. Der Autoklav wurde entspannt und das Reaktionsgemisch gaschromatographisch entsprechend Beispiel 1.1 analysiert. Es wurde ein Verhältnis Edukt/Cyanessigsäureethylester von von 3: 1 gefunden.

Die Ergebnisse der vorstehenden Beispiele sind in Tabelle 1 zusammengefasst.

**Tabelle 1:**

| **Beispiel** | **R =** | **Kat.** **[mol-%]** | **NHPI** **[mol-%]** | **Lsm.** | **ϑ [°C]/t [h]** | **Edukt/Produkt** |
|---|---|---|---|---|---|---|
| 1.1 | Me | 0,65 | 8,6 | Essigsäure | 150 / 5 | 11:1 |
| 1.2 | Me | 1,3 | 17,5 | Acetonitril | 190 / 7 | 2:1 |
| 1.3 | Me | 0,17 | 2,6 | ― | 130 / 3 | 9:1 |
| 1.4 | Me | 2,9 | 21,8 | Acetonitril | 180 / 5,5 | 5:1 |
| 2 | Et | 1,5 | 20,3 | Acetonitril | 190 / 7 | 3:1 |
| Abkürzungen: Me = Methyl, Et = Ethyl, Kat. = Katalysator, NHPI = *N*-Hydroxyphthalimid, Lsm. = Lösungsmittel | | | | | | |

## Patentansprüche

1. Verfahren zur Herstellung von Cyanessigsäureestern der allgemeinen Formel 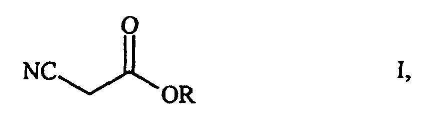 worin R eine lineare oder verzweigte C₁₋₈-Alkylgruppe bedeutet, **dadurch gekennzeichnet, dass** ein Alkoxypropionitril der allgemeinen Formel 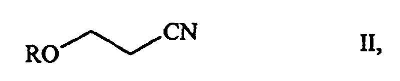 worin R die oben genannte Bedeutung hat, in Gegenwart einer Cobaltverbindung als Katalysator mit Sauerstoff oder einem Sauerstoff bildenden Reagens direkt zu (I) oxidiert wird.

2. Verfahren nach Anspruch 1, **dadurch gekennzeichnet, dass** als Cobaltverbindung Cobalt(II)acetat-tetrahydrat oder Cobaltacetylacetonat eingesetzt wird.

3. Verfahren nach einem der Ansprüche 1 bis 2, **dadurch gekennzeichnet, dass** der Katalysator in einer Menge von 0,01 bis 10 mol %, bezogen auf das Alkoxypropionitril (II), eingesetzt wird.

4. Verfahren nach einem oder mehreren der Ansprüche 1 bis 3, **dadurch gekennzeichnet, dass** die Umsetzung bei einer Temperatur von 50 bis 250 °C durchgeführt wird.

5. Verfahren nach einem oder mehreren der Ansprüche 1 bis 4, **dadurch gekennzeichnet, dass** die Umsetzung in einem organischen Lösungsmittel durchgeführt wird.

## Claims

1. Process for the preparation of cyanoacetic acid esters of the general formula 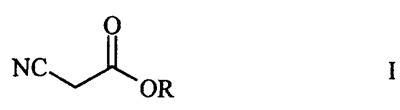 in which R is a linear or branched C₁₋₈-alkyl group, **characterized in that** an alkoxypropionitrile of the general formula 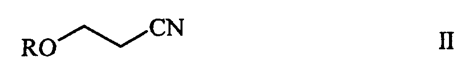 in which R has the meaning given above is directly oxidized to (I) using oxygen or an oxygen-forming reagent in the presence of a cobalt compound as catalyst.

2. Process according to Claim 1, **characterized in that** the cobalt compound used is cobalt(II) acetate tetrahydrate or cobalt acetylacetonate.

3. Process according to one of Claims 1 to 2, **characterized in that** the catalyst is employed in an amount from 0.01 to 10 mol%, relative to the alkoxypropionitrile (II).

4. Process according to at least one of Claims 1 to 3, **characterized in that** the reaction is carried out at a temperature of 50 to 250 °C.

5. Process according to at least one of Claims 1 to 4, **characterized in that** the reaction is carried out in an organic solvent.

## Revendications

1. Procédé pour la préparation d'esters d'acide cyanoacétique de formule générale 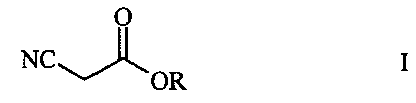 dans laquelle R représente un groupe alkyle en C₁₋₈ linéaire ou ramifié, **caractérisé en ce qu'**un alcoxypropionitrile de formule générale 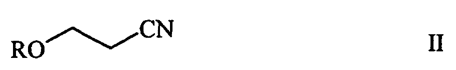 dans laquelle R a la signification donnée ci-dessus, est oxydé directement en (I) par de l'oxygène ou un réactif générateur d'oxygène, en présence d'un composé contenant du cobalt en tant que catalyseur.

2. Procédé selon la revendication 1, **caractérisé en ce qu'**on utilise en tant que composé contenant du cobalt de l'acétate de cobalt-(II) tétrahydraté ou de l'acétylacétonate de cobalt.

3. Procédé selon la revendication 1 ou 2, **caractérisé en ce que** le catalyseur est utilisé en une quantité de 0,01 à 10 % en moles, par rapport à l'alcoxypropionitrile (II).

4. Procédé selon une ou plusieurs des revendications 1 à 3, **caractérisé en ce que** la réaction est effectuée à une température de 50 à 250 °C.

5. Procédé selon une ou plusieurs des revendications 1 à 4, **caractérisé en ce que** la réaction est effectuée dans un solvant organique.
